# EUROPEAN PATENT SPECIFICATION

(11) **EP 1 748 764 B1**
(45) Date of publication and mention of the grant of the patent: **30.04.2008**
(21) Application number: 05731323.1
(22) Date of filing: 15.04.2005
(51) Int. Cl.: A61K 9/50, A61K 9/16, A61K 31/135

(54) **AN AMINE DRUG-CONTAINING SLOW-RELEASE GRANULE PREPARATION BASED ON PARTICLES WITH A COATING LAYER AND THE CORRESPONDING METHOD OF PRODUCTION**
EIN AMIN-ARZNEIMITTEL ENTHALTENDE GRANULATZUBEREITUNG MIT LANGSAMER FREISETZUNG AUF BASIS VON TEILCHEN MIT EINER ÜBERZUGSSCHICHT UND ENTSPRECHENDES HERSTELLUNGSVERFAHREN
Préparations contenant des granules avec un agent de la structure d'amine à libération lente - basées aux particules enrobés et leur procédé de production

(30) Priority: 21.04.2004 JP 2004125550
(43) Date of publication of application: 07.02.2007
(73) Proprietor: BASF SE, 67056 Ludwigshafen (DE)
(72) Inventor: TANABE, Mitsunori, Senboku-gun, Osaka-fu (JP); TANAKA, Tadaaki, Chiba-ken (JP); YOKOTA, Kunihiko, Suita-shi, Osaka-fu (JP); KAKIGUCHI, Yoshitomi, Kawabe-gun, Hyogo-ken (JP); KAJIURA, Tomoyoshi, Yokkaichi-shi, Mie-ken (JP)
(86) International application number: PCT/EP2005/003998
(87) International publication number: WO 2005/102269

(56) References cited:
- WO-A-20/04038428
- US-A1- 2001 007 680
- US-A1- 2003 175 342
- US-B1- 6 419 954

## Description

The present inventions relate to slow-release preparations in which the effective ingredient is an amine drug; and to a method for the production thereof

In US6419954 the tablet comprises a gel-forming material and at least one particle comprising an active agent (which may be an amine derivative amongst others) in contact with a coating material to modify release of the active agent. The coating material is based on a polymer (polyvinyl acetate amongst other possibilities) and a plasticizer, a stabilizer or both.

In US2001/007680 a film-coating is described consisting of polyvinyl acetate, hydrophilic additives, other coating ingredients, which serves as a taste-masking coating for oral dosage forms. The core is composed of an active ingredient (which may be an amine derivative), a binder, an emulsifier and disintegrant.

US2001/007680 discloses a pharmaceutical single unit administration form provided with a sealed film coating and having delayed release of an active compound, the film-coating comprising polyvinyl acetate as film-forming agent. The coating may consist of two or more layers.

WO2004/038428 discloses a solid dosage form comprising a) a core (active agent (pseudoephedrine), binder) dispersed in a first release-controlling matrix and b) a coat including the drug in a second release-controlling matrix.

Amine drugs such as pseudoephedrine hydrochloride and phenylpropanolamine hydrochloride are valuable as the effective ingredient of oral drugs for rhinitis (see, for example, Patent Reference 1, JP-A-2003-300874). Furthermore, as coating agents for slow-release preparations, aqueous slow-release coating agents comprising ethyl cellulose aqueous dispersions or ethyl acrylate/methyl methacrylate copolymer aqueous dispersions are normally used (see, for example, Patent Reference 2, JP-A-09-71524).

However, it is difficult to fully control dissolution when amine drugs such as pseudoephedrine hydrochloride or phenylpropanolamine hydrochloride are given a coating treatment using an ethyl cellulose aqueous dispersion or an ethyl acrylate/methyl methacrylate copolymer aqueous dispersion and, moreover, in some cases aggregation occurs during the coating operation and the operation itself becomes difficult.

The present inventions have the objective of providing drug preparations where the dissolution of an amine drug can be effectively controlled.

The present inventions encompass the following.
(1) Slow-release granules formed by providing a coating layer containing polyvinyl acetate and surfactant on drug-containing particles comprising an amine drug and a water-soluble binder coated onto the surface of a core material.
(2) Slow-release granules according to (1) above where the amine drug is of at least one kind selected from pseudoephedrine, phenylpropanolamine and their acid addition salts.
(3) Slow-release granules according to (1) or (2) above where the surfactant is sodium lauryl sulphate.
(4) Slow-release granules according to any of (1) to (3) above where the coating layer contains polyvinyl pyrrolidone.
(5) Slow-release granules according to any of (1) to (4) above where the coating layer contains plasticizer.
(6) A method for the production of slow-release granules which includes
   (i) a stage in which a liquid comprising an amine drug and a water-soluble binder dissolved or dispersed in an aqueous solvent is sprayed onto the surface of a core material and dried to form a drug layer, and
   (ii) a stage in which an aqueous coating liquid containing polyvinyl acetate and surfactant is sprayed onto the drug-containing particles obtained in stage (i) and dried to form a coating layer
(7) A method according to (6) above where the coating liquid contains polyvinyl pyrrolidone.
(8) A method according to (6) or (7) above where the coating liquid contains plasticizer.
(9) Slow-release granules produced by a method as described in any of (6) to (8) above.
(10) Capsules or tablets which contain slow-release granules according to any of (1) to (5) and (9) above.
(11) A drug preparation storage method which is characterized in that slow-release granules according to any of Claims (1) to (5) and (9) or the capsules or tablets according to (10) are stored at a relative humidity of no more than 43%.

In accordance with the present inventions, it is possible to provide drug preparations where the dissolution of an amine drug can be effectively controlled.

The inventive slow-release granules are formed by providing a coating layer containing polyvinyl acetate and surfactant on drug-containing particles comprising an amine drug and a water-soluble binder coated onto the surface of a core material.

In the inventions, slow-release granules refers to the coarse granules and fine granules specified in the 12^{th} Revised Japanese Pharmacopoeia, where the amine drug, which comprises the effective ingredient, is dissolved out over at least 1 hour.

The amine drugs employed in the present inventions are not restricted and may be primary amines, secondary amines, tertiary amines or their acid addition salts, and they may be used on their own or in combinations of two or more. Now, amide compounds which do not form acid addition salts, such as acetaminophen, are not included amongst the amine drugs.

Examples of the amine drugs are pseudoephedrine, phenylpropanolamine, phenylephrine, methylephedrine, chlorpheniramine, diphenhydramine, methoxy-phenamine and dopamine, and as examples of their acid addition salts there are the hydrochloride, sulphate, nitrate, iodide and other inorganic acid salts, and the citrate, maleate, acetate, tartrate, salicylate, tannate and other organic acid salts. There are no special restrictions thereon providing they meet the (import) approval standards for the production of pharmaceutical products such as cold remedies, oral drugs for rhinitis, antitussives/ expectorants and the like.

The core material used in the present inventions is not particularly restricted providing it is a pharmaceutical additive but preferred examples are crystalline cellulose particles (such as Avicel SP, commercial name of an Asahi Chemical Industry Co. product; and Selfia CP, commercial name of an Asahi Chemical Industry Co. product), white sugar particles (such as Nonpareil 103, commercial name of a Freund Industrial Co. product) and white sugar/starch particles (such as Nonpareil 101, commercial name of a Freund Industrial Co. product), etc, of particle diameter 150-750 µm (preferably 300-500 µm).

Examples of the water-soluble binder used in the drug layer are copolyvidone, polyvinyl pyrrolidone, polyethylene glycol-polyvinyl alcohol graft polymer, polyvinyl alcohol, cellulose derivatives (such as hydroxypropyl methyl cellulose, hydroxypropyl cellulose, methyl cellulose) and the like.

The proportions of core material and amine drug by weight are normally 100 : 1 to 100 : 200, and preferably 100 : 5 to 100 : 100; and the proportions of core material and water-soluble binder by weight are normally 100 : 0.5 to 100 : 25, and preferably 100 : 4 to 100 : 10.

The proportions of amine drug and water soluble binder by weight are normally 20 : 1 to 1 : 1, and preferably 10 : 1 to 2 : 1.

As well as the amine drug and the water-soluble binder, there may be suitably added to the drug layer, within a range that does not impair the efficacy of the invention, lubricants (such as magnesium stearate and sucrose fatty acid esters) and fluidizing agents (such as talc or silicon dioxide).

Examples of the surfactant used in forming the coating layer are sodium lauryl sulphate and Tween 80. The proportions of polyvinyl acetate and surfactant by weight are normally 100 : 0.1 to 100 : 10, and preferably 100 : 0.5 to 100 : 2.

The coating layer preferably contains polyvinyl pyrrolidone. The proportions of the polyvinyl acetate and polyvinyl pyrrolidone by weight are normally 100 : 1 to 100 : 30, and preferably 100 : 5 to 100 : 20.

If a compatible plasticizer is included in the coating liquid used to form the coating layer, the slow release effect is further enhanced. Examples of such plasticizers are triethyl citrate, Triacetin, propylene glycol, polyethylene glycol, tributyl acetylcitrate and 2-pyrrolidone, preferably triethyl citrate and Triacetin. The plasticizer content (by solids component) in the coating layer in the case of triethyl citrate, Triacetin, tributyl acetylcitrate and 2-pyrrolidone is preferably 2 to 10 wt%, and in the case of propylene glycol and polyethylene glycol it is preferably 5 to 20 wt%. In the case of triethyl citrate and Triacetin, from 2.5 to 7.5 wt% is particularly preferred.

Furthermore, a coating auxiliary (such as talc or silicon dioxide) may be suitably added to the coating liquid.

The inventive slow release granules are preferably produced by the following stages,
(i) a stage in which a liquid comprising the amine drug and water-soluble binder dissolved or dispersed in an aqueous solvent is sprayed onto the surface of a core material and dried to form the drug layer, and
(ii) a stage in which an aqueous coating liquid containing polyvinyl acetate and surfactant is sprayed onto the drug-containing particles obtained in stage (i) and dried to form a coating layer, and the coating layer stabilized without curing or by carrying out curing at no more than 60°C, preferably 40-60°C.

Polyvinyl acetate (vinyl acetate resin) does not dissolve in water (see page 319 of "Pharmaceutical Additives Standards 2003" published by Yakuji Nipposha) so, in the use thereof as a coating agent, it has normally been necessary to employ an organic solvent. However, in accordance with the present inventions it is possible to produce slow-release granules without using an organic solvent.

Normally, water is employed as the aqueous solvent in aforesaid stage (i), but water may also be employed in which there is mixed a solvent which is readily miscible with water (such as an alcohol).

The proportions of the aqueous solvent and amine drug by weight are normally 100 : 5 to 100 : 60, preferably 100 : 15 to 100 : 40, and the proportions of the aqueous solvent and water-soluble binder by weight are normally 100 : 0.2 to 100 : 60, preferably 100 : 1.5 to 100 : 20.

The solvent in the aqueous coating liquid used in aforesaid stage (ii) is normally water but the water may also be mixed with a small amount of a solvent which is readily miscible with water (such as an alcohol).

The concentration of the polyvinyl acetate in the aqueous coating liquid is normally 5 to 27 wt% and preferably 12 to 23 wt%, and the concentration of surfactant (preferably sodium lauryl sulphate) is normally 0.05 to 0.4 wt% and preferably 0.1 to 0.35 wt%.

The amount of the aqueous coating liquid applied will differ depending on the desired dissolution time but the percentage polyvinyl acetate in said aqueous coating liquid in terms of the weight of drug-containing particles is normally 5 to 100 wt% and preferably 10 to 60 wt%. Moreover, the percentage by weight of the total solids component in said aqueous coating liquid in terms of the weight of drug-containing particles (hereinafter referred to as the percentage coating) is normally 10 to 100% and preferably 20 to 60%.

Next, explanation is provided of a preferred mode in the case of the inventive method for producing slow-release granules. Firstly, the core material is introduced into a tumbling fluidized bed coating machine, and heated air then blown-in, and tumbling and fluidizing effected. The drug liquid formed by dissolving or dispersing the amine drug and water-soluble binder is sprayed thereon and dried, to form the drug layer. Next, the dried drug-containing particles are introduced into a tumbling fluidized bed coating machine and, while tumbling and fluidizing are effected by blowing-in heated air, the aforesaid aqueous coating liquid is sprayed thereon and slow-release coating carried out. Next, drying is performed, and the inventive slow-release granules obtained.

The inventive slow-release granules obtained in this way can optionally be mixed with other drugs and the like, and employed in the form of capsules or tablets.

By storing the inventive slow-release granules, and capsules or tablets employing same, at a relative humidity of no more than 43%, it is possible to maintain the same dissolution characteristics as prior to storage.

### Examples

Below, the inventions are explained in more specific terms by means of examples and comparative examples but the inventions are not to be restricted to these examples.

### (Example 1)

### 1. Formation of the drug layer

2425 g of pseudoephedrine hydrochloride and 485 g of copolyvidone (commercial name Kollidon VA64, produced by BASF) were dissolved in 8728 g of pure water to prepare the drug liquid. Furthermore, 21.3 kg of cellulose granules of particle diameter 350-500 µm (Seffia CP-305, produced by the Asahi Chemical Industry Co.) were introduced into a tumbling fluidized bed coating machine (model MP-25, produced by the Powrex Corporation). Using a tangential spray, the drug liquid was sprayed onto the fluidized cellulose granules and a drug layer formed. The operating conditions were as follows:-

| | |
|---|---|
| air supply temperature | = 75°C |
| air supply flow rate | = 8.0 m³/min |
| exhaust air temperature | = 41-42°C |
| spray rate | = 100 mL/min |
| spray air volume | = 450 L/min |
| side air pressure | = 400 L/min |
| rotation rate | = 240 rpm |
| drying time | = 10 min |

### 2. Slow-release coating

850 g of a BASF aqueous slow-release coating agent "polyvinyl acetate aqueous dis-person" (commercial name Kollicoat SR30D) [containing 27 wt% polyvinyl acetate, 2.5 wt% polyvinyl pyrrolidone, 0.3 wt% sodium lauryl sulphate; solids concentration 30 wt%], 15 g of plasticizer (one type selected from Triacetin, triethyl citrate, propylene glycol and polyethylene glycol 400), 30 g of talc and 850 g of pure water were dispersed using a stirrer, and a coating liquid prepared. Furthermore, 1000 g of the drug-containing granules prepared by the aforesaid method were introduced into a tumbling fluidized bed coating machine (model MP-01, produced by the Powrex Corporation). The coating liquid was then sprayed onto the fluidized granules using a tangential spray, and slow-release coating performed. After completing the coating, preliminary drying was carried out, after which the product was removed and curing carried out for 12 hours under vacuum at 40°C. The operational conditions were as follows:-

| | |
|---|---|
| amount introduced | = 1,000 g |
| air supply temperature | = 50°C |
| air supply rate | = 70 m³/hr |
| exhaust air temperature | = 30-33°C |
| spray rate | = 8-10 g/min |
| spray air volume | = 70 Umin |
| rotation rate | = 350 rpm |
| preliminary drying time | = 5 min |

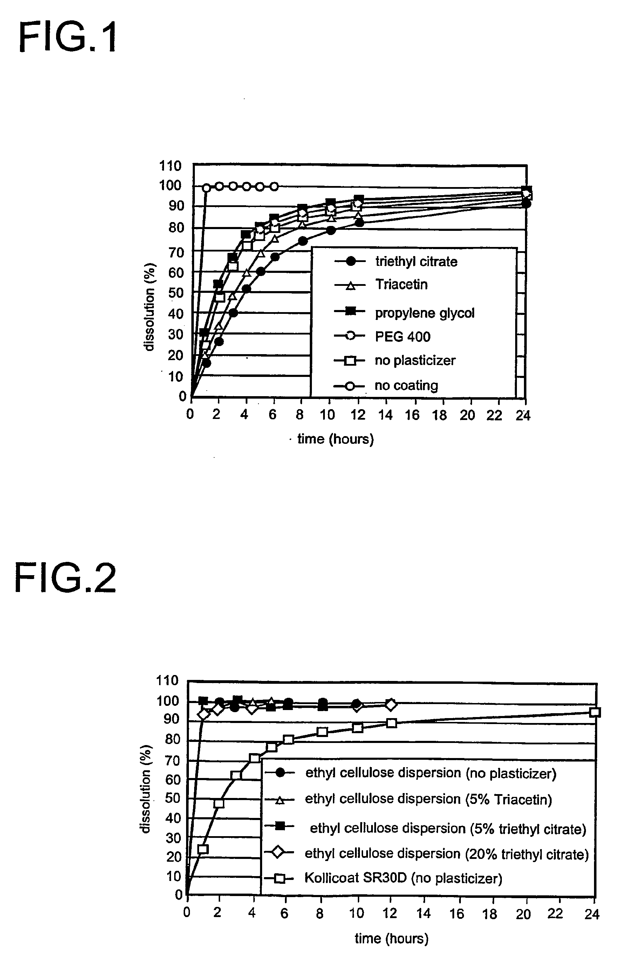

The dissolution characteristics of the granules obtained were investigated by Method 2 of the Japanese Pharmacopoeia. Liquid 1 (pH =1.2) was used as the test liquid and the rotation rate of the paddle was 50 rpm. The results are shown in Figure 1.

In the case where no coating was carried out, there was total dissolution within 10 minutes. In contrast, by coating with Kollicoat SR30D, slow release could be achieved. Furthermore, the addition of Triacetin or triethyl citrate further enhanced the slow release effect.

### (Comparative Example 1)

Testing was carried out in the same way as in Example 1 using an ethyl cellulose aqueous dispersion (commercial name Aquacoat ECD, produced by the FMC Co.) instead of the Kollicoat SR30. In this case there was no dissolution controlling effect, and even when the plasticizer was increased to 20% no effect of the addition was apparent. The results are shown in Figure 2. Furthermore, testing was also carried out in the same way using an ethyl acrylate/methyl methacrylate copolymer aqueous dispersion (commercial name Eudragit NE30D, produced by the Rohm Co., or Kollicoat EMM30D, produced by BASF) instead of the Kollicoat SR30. Here, aggregation occurred during the coating operation, so the coating operation was halted.

### (Example 2)

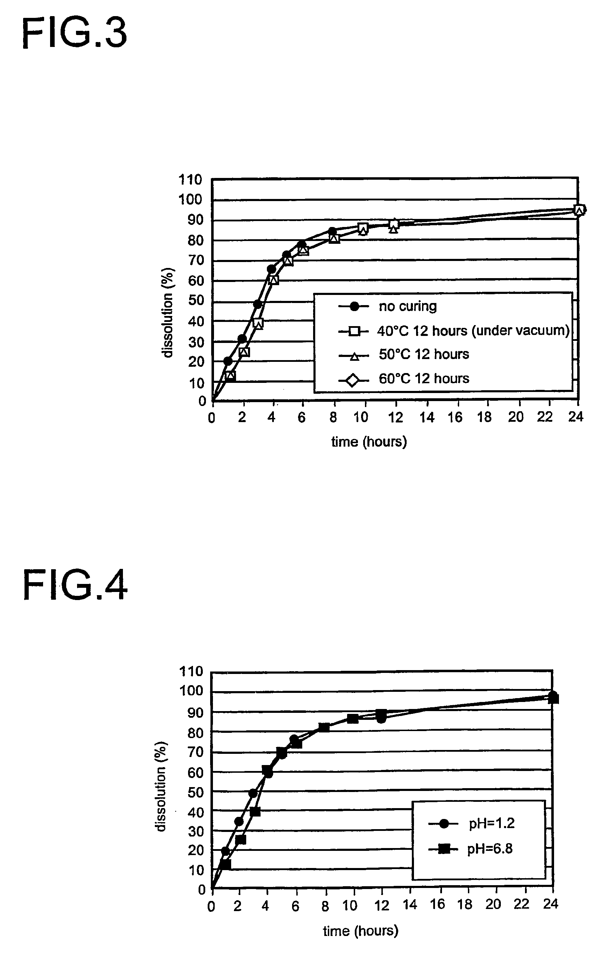

Slow-release coating was carried out under the same conditions as in Example 1 and, after preliminary drying and removal of the product, curing was carried out for 12 hours at 50°C or 60°C. An investigation was then carried out into the dissolution properties of the granules obtained (employing Triacetin as plasticizer). It was found that the dissolution behaviour after carrying out drying for 12 hours at 50°C or 60°C was the same as in the case of drying under vacuum at 40°C. The results are shown in Figure 3.

### (Example 3)

Using Liquid 2 (pH = 6.8), a dissolution test was carried out under conditions identical to those in Example 1 (employing Triacetin as plasticizer). It was found, as a result, that the same dissolution behaviour was again shown with this Liquid 2. The results are shown in Figure 4.

### (Example 4)

A drug liquid was prepared by dissolving 375 g of pseudoephedrine hydrochloride and 75 g of copolyvidone (commercial name Kollidon VA64, produced by BASF) in 2166 g of pure water. Furthermore, 800 g of cellulose granules of particle diameter 350-500 µm (Selfia CP-305, produced by the Asahi Chemical Industry Co.) were introduced into a tumbling fluidized bed coating machine (model MP-01, produced by the Powrex Corporation). The drug liquid was then sprayed onto the fluidized cellulose granules to form the drug layer. The operational conditions were as follows:-

| | |
|---|---|
| air supply temperature | = 75°C |
| air supply rate | = 42 m³/hr |
| exhaust air temperature | = 41-42°C |
| spray rate | = 8.5-10 mL/min |
| spray air volume | = 60 Umin |
| rotation rate | = 300 rpm |
| drying time | = 10 min |

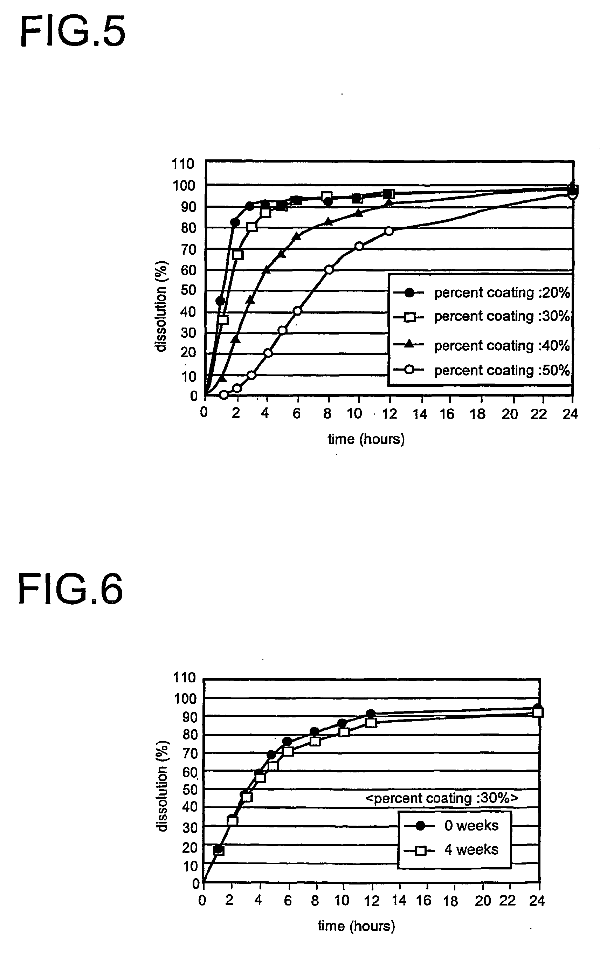

875 g of the aqueous slow-release coating agent "polyvinyl acetate aqueous suspension" produced by BASF (commercial name Kollicoat SR30D) [containing 27 wt% polyvinyl acetate, 2.5 wt% polyvinyl pyrrolidone and 0.3 wt% sodium lauryl sulphate; solids concentration 30 wt%], 7.5 g of Triacetin, 30 g of talc and 833 g of pure water were dispersed using a stirrer, to prepare a coating liquid. Furthermore, 1000 g of drug-containing granules prepared by the method in Example 1 ["1. Formation of the drug layer"] were introduced into a tumbling fluidized bed coating machine (model MP-01, produced by the Powrex Corporation). The coating liquid was then sprayed onto the fluidized granules by means of a tangential spray and slow release coating carried out. The percentage coating in this case was 30%. Furthermore, coating at levels of 40% and 50% was also carried out in the same way. The operational conditions were the same as in Example 1. The dissolution characteristics of the granules obtained were determined under the same conditions as in Example 1. The results are shown in Figure 5. A slow release effect was possible by coating with the aforesaid coating agent at a weight of 30% or more.

### (Example 5)

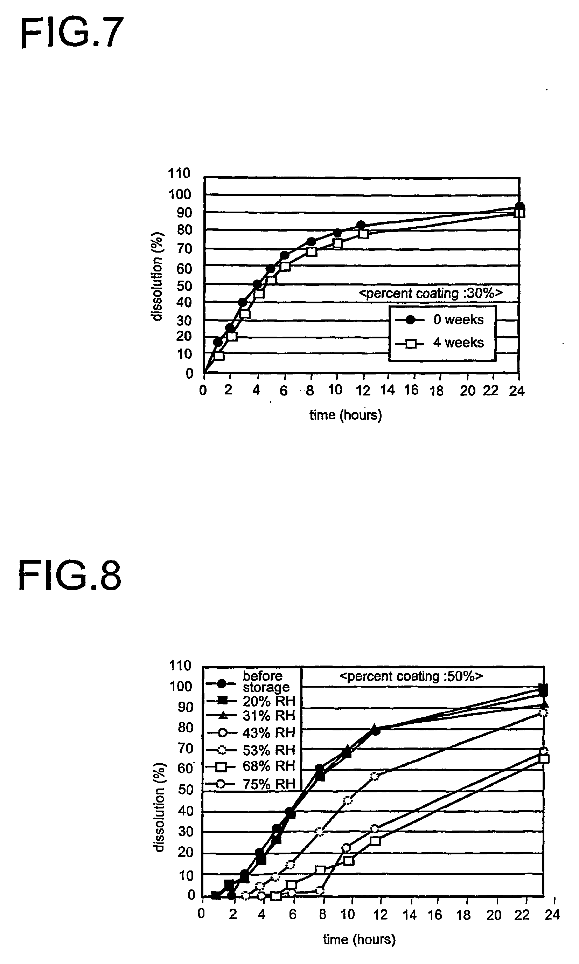

Granules prepared by the method in Example 1 (using Triacetin or triethyl citrate as a plasticizer) were placed in a glass bottle and a stopper inserted, after which they were stored for 4 weeks at 40°C. The dissolution of the sample following storage was found to be about the same as that prior to storage. Figure 6 shows the results when Triacetin was used and Figure 7 shows the results when triethyl citrate was used as the plasticizer, respectively.

### (Example 6)

The granules prepared in Example 4 (percentage coating 50%) were introduced into glass bottles and stored for 4 weeks at 40°C and at a relative humidity of 20%, 31%, 43%, 53%, 68% or 75%. The results are shown in Figure 8.

When stored at 20%, 31% or 43% relative humidity, the dissolution characteristics after storage were the same as those prior to storage. In contrast, when stored at 53%, 68% or 75% relative humidity, the dissolution was slowed.

### Brief Explanation of the Drawings

[Figure 1] This shows the pseudoephedrine dissolution characteristics of granules containing pseudoephedrine hydrochloride in the case where plasticizer was added (5 wt% solids content), or not added, to an aqueous coating liquid containing polyvinyl acetate, polyvinyl pyrrolidone and sodium lauryl sulphate. The percentage coating was 30%.

[Figure 2] This shows the pseudoephedrine dissolution characteristics of granules containing pseudoephedrine hydrochloride in the case where plasticizer was added (5 wt% or 20 wt% solids content), or not added, to an ethyl cellulose aqueous dispersion. The percentage coating was 30%.

[Figure 3] This shows the influence of curing temperature on the dissolution characteristics of the pseudoephedrine hydrochloride. The percentage coating was 30%.

[Figure 4] This shows the influence of pH on the dissolution characteristics of the pseudoephedrine hydrochloride. The percentage coating was 30%.

[Figure 5] This shows the influence of percentage coating on the dissolution characteristics of the pseudoephedrine hydrochloride; 37°C, 50 rpm, pH = 1.2

[Figure 6] This shows the pseudoephedrine dissolution characteristics after 4 weeks at 40°C in the case of granules containing pseudoephedrine hydrochloride which had been coated with an aqueous coating liquid containing polyvinyl acetate, polyvinyl pyrrolidone and sodium lauryl sulphate, together with Triacetin as plasticizer. The percentage coating was 30%.

[Figure 7] This shows the pseudoephedrine dissolution characteristics after 4 weeks at 40°C in the case of granules containing pseudoephedrine hydrochloride which had been coated with an aqueous coating liquid containing polyvinyl acetate, polyvinyl pyrrolidone and sodium lauryl sulphate, together with triethyl citrate as plasticizer. The percentage coating was 30%.

[Figure 8] This shows the influence of the relative humidity at the time of storage on the dissolution characteristics of pseudoephedrine hydrochloride. The amount of plasticizer (Triacetin) was 5 wt% based on solids content.

## Claims

1. Slow-release granules formed by providing a coating layer containing polyvinyl acetate and surfactant on drug-containing particles comprising an amine drug and a watet-solubie binder coated onto the surface of a core material.

2. Slow-release granules according to Claim 1 where the amine drug is of at least one kind selected from pseudoephedrine, phenylpropanolamine and their acid addition salts.

3. Slow-release granules according to Claims 1 or 2 where the surfactant is sodium lauryl sulphate.

4. Slow-release granules according to any of Claims 1 to 3 where the coating layer contains polyvinyl pyrrolidone.

5. Slow-release granules according to any of Claims 1 to 4 where the coating layer contains plasticizer.

6. A method for the production of slow-release granules which includes
(i) a stage in which a liquid comprising an amine drug and a water-soluble binder dissolved or dispersed in an aqueous solvent is sprayed onto the surface of a core material and dried to form a drug layer, and
(ii) a stage in which an aqueous coating liquid containing polyvinyl acetate and surfactant is sprayed onto the drug-containing particles obtained in stag (i) and dried to form a coating layer.

7. A method according to Claim 6 where the coating liquid contains polyvinyl pyrrolidone.

8. A according to Claim 6 or 7 where the coating liquid contains plasticizer.

9. Slow-release granules obtainable by a method as described in any of Claims 6 to 8.

10. Capsules or tablets which contain slow-release granules according to any of Claims 1 to 5 and 9.

## Patentansprüche

1. Granulat mit langsamer Freisetzung, gebildet durch Bereitstellung einer Polyvinylacetat und Tensid enthaltenden Überzugsschicht auf arzneimittelhaltigen Partikeln, die ein Amin-Arzneimittel und ein wasserlösliches Bindemittel aufgetragen auf die Oberfläche eines Kernmaterials enthalten.

2. Granulat mit langsamer Freisetzung nach Anspruch 1, wobei es sich bei dem Amin-Arzneimittel um wenigstens ein Arzneimittel ausgewählt aus Pseudoephedrin, Phenylpropanolamin und ihren Säureadditionssalzen handelt.

3. Granulat mit langsamer Freisetzung nach Anspruch 1 oder 2, wobei es sich bei dem Tensid um Natriumlaurylsulfat handelt.

4. Granulat mit langsamer Freisetzung nach einem der Ansprüche 1 bis 3, wobei die Überzugsschicht Polyvinylpyrrolidon enthält.

5. Granulat mit langsamer Freisetzung nach einem der Ansprüche 1 bis 4, wobei die Überzugsschicht Weichmacher enthält.

6. Verfahren zur Herstellung eines Granulats mit langsamer Freisetzung, umfassend
(i) eine Stufe, bei der man eine Flüssigkeit, die ein Amin-Arzneimittel und ein wasserlösliches Bindemittel gelöst oder dispergiert in einem wäßrigen Lösungsmittel umfaßt, auf die Oberfläche eines Kernmaterials sprüht und unter Bildung einer Arzneimittelschicht trocknet und
(ii) eine Stufe, bei der man eine wäßrige Überzugsflüssigkeit, die Polyvinylacetat und Tensid enthält, auf die in Stufe (i) erhaltenen arzneimittelhaltigen Partikel sprüht und unter Bildung einer Überzugsschicht trocknet.

7. Verfahren nach Anspruch 6, wobei die Überzugsflüssigkeit Polyvinylpyrrolidon enthält.

8. Verfahren nach Anspruch 6 oder 7, wobei die Überzugsflüssigkeit Weichmacher enthält.

9. Granulat mit langsamer Freisetzung, erhältlich durch ein wie in einem der Ansprüche 6 bis 8 beschriebenes Verfahren.

10. Kapseln oder Tabletten, enthaltend ein Granulat mit langsamer Freisetzung nach einem der Ansprüche 1 bis 5 und 9.

## Revendications

1. Granules à libération lente formées en rappliquant une couche d'enrobage contenant de l'acétate de polyvinyle et un tensioactif sur des particules contenant le médicament comprenant un médicament amine et un liant hydrosoluble venant recouvrir la surface d'un matériau noyau.

2. Granules à libération lente selon la revendications 1, où le médicament amine est d'au moins un type sélectionne parmi la pseudoéphédrine, la phénylpropanolamine et leurs sels d'addition d'acide.

3. Granules à libération lente selon la revendications 1 ou 2, où le tensioactif est du sulfate laurique de sodium.

4. Granules à libération lente selon une des revendications de 1 à 3, où la couche d'enrobage contient du polyvinylpyrrolidone.

5. Granules à libération lente selon une des revendications de 1 à 4, où la couche d'enrobage contient du plastifiant.

6. Procédé de production de granules à libération lente qui inclut :
(i) une étape dans laquelle un liquide comprenant un médicament amine et un liant hydrosoluble dissous ou dispersé dans un solvant aqueux est pulvérisé sur la surface d'un matériau noyau et est séché pour former une couche de médicament; et
(ii) une étape dans laquelle un liquide d'enrobage aqueux contenant de l'acétate de polyvinyle et un tensioactif est pulvérisé sur les particules contenant le médicament qui sont obtenues dans l'étape (i) et est séché pour former une couche d'enrobage.

7. Procédé selon la revendications 6, où le liquide d'enrobage contient du polyvinylpyrrolidone.

8. Procédé selon la revendication 6 ou 7, où le liquide d'enrobage contient du plastifiant.

9. Granules à libération lente susceptibles d'être obtenus par un procédé tel que celui décrit dans une des revendications de 6 à 8.

10. Capsules ou comprimés qui contiennent des granules à libération lente selon une des revendications de 1 à 5 et la revendication 9.
